# EUROPEAN PATENT SPECIFICATION

(11) **EP 2 024 761 B1**
(45) Date of publication and mention of the grant of the patent: **07.05.2014**
(21) Application number: 07711694.5
(22) Date of filing: 27.02.2007
(51) Int. Cl.: G01T 1/161

(54) **METHOD AND DEVICE FOR 3D ACQUISITION, 3D VISUALIZATION AND COMPUTER GUIDED SURGERY USING NUCLEAR PROBES**
VERFAHREN UND EINRICHTUNG ZUR 3D-BESCHAFFUNG, 3D-VISUALISIERUNG UND COMPUTERGEFÜHRTEN CHIRURGIE UNTER VERWENDUNG VON NUKLEARSONDEN
PROCÉDÉ ET DISPOSITIF D'ACQUISITION 3D, DE VISUALISATION 3D ET DE CHIRURGIE GUIDÉE PAR ORDINATEUR AU MOYEN DE SONDES NUCLÉAIRES

(30) Priority: 16.05.2006 EP 06010052
(43) Date of publication of application: 18.02.2009
(73) Proprietor: SurgicEye GmbH, 81671 München (DE)
(72) Inventor: NAVAB, Nassir, 81247 München (DE); ZIEGLER, Sibylle, 80797 München (DE); TRAUB, Jörg, 81371 München (DE); WENDLER, Thomas, 81247 München (DE)
(74) Representative: Zimmermann & Partner
(86) International application number: PCT/EP2007/001678
(87) International publication number: WO 2007/131561

(56) References cited:
- WO-A-01/89384
- US-A- 6 167 296
- US-A1- 2004 204 646

## Description

The present invention relates to a method and to an device for 3D acquisition, 3D visualization and computer guided surgery using Nuclear Probes with a tracking system and a to localize malign tumorous cells of a human body or animal and a surgical instrument to remove the said diseased cells in both invasive and minimally invasive surgery.

The prior art related to the present invention are disclosed for example in US 6,602,488, US 6,456,869, US 6,317,622 or US 6,167,296 and allow trailing of the hand held probes as common diagnostic devices especially during surgery, as well as tracking systems for determination of position and orientation of surgical instruments and imaging devices. Furthermore surface reconstruction is known as part of the state of the art.
The idea of tracking nuclear probes was mentioned in the past by several groups for example as disclosed in US 6,510,336 and US 6021341. However, these patents did not provide neither theory nor implementation nor application.
As further disclosed in US 6,643,538 nuclear probes can be integrated per construction with a camera. However, this patent does not consider any kind of spatial localization.

US2004204646 describes an intracorporeal-imaging head, which combines at least optical and radioactive-emission imaging, possibly also with high-resolution position tracking. The radioactive-emission-imaging probe has a wide-aperture, or coarse collimator. The intracorporeal-imaging head may further include ultrasound and MRI imagers, as well as a surgical instrument.

Intraoperative probes have been used in the localization of tumors for 60 years. Lately beta emitting labeling has improved drastically their detection accuracy enabling a minimal, but complete resection of malignant cells and thus avoiding recurrence.

The output of nuclear probes is just a one dimensional signal usually not constant in time. The main advantages of such devices lie in the portability, simplicity, and the possibility of miniaturizing them for the investigation of cavities for instance mounted on endoscopes. Moreover since each measurement is not restricted to be at a certain position with respect to the previous one, probes allow the scan of arbitrary surfaces with a spatial accuracy only limited by the size of the sensor.
Nuclear probes such as gamma- and beta-probes are capable of measuring radioactive decay of nuclides in tracers that are injected to the patient before the intervention.
The combination of nuclear probes with a camera in one device allows them further not only the determination of the radioactivity emitted by a certain region, but also the simultaneous visualization of the anatomy. This even allows the use of these nuclear probes to detect lesions through smaller incisions where the point of emission is only partially visible or completely occluded to the surgeon.

The disadvantage of these nuclear probes is that they are just point measurements. This makes the appreciation of the physical value on a surface difficult if it changes considerably with position. One additional problem in this matter is the fluctuation of the measurement results which is based on the statistical nature of the decay process which makes the interpretation of the measurement data even more difficult and unreliable. A further disadvantage is the need for many observations in order to get an idea of a valid measurement map in big areas, which is not the best solution for detection of hot spots in scans of big body sections.
Moreover, the sequential process of measuring and then performing the surgical action limits the accuracy to the surgeon's ability of navigating the instrument back to the detected locations. Finally, in the specific case of the combination of nuclear probes with a camera one has a further disadvantage that the correlation of the measurement of the radioactivity to the anatomy seen in the video image is not given hence it has to be done in the mind of the surgeon.

The advantageous extending of the use of probes for combining position and orientation tracking with surface reconstruction, measurement and advanced visualization is not mentioned in the past.

The object of the present invention is to improve a computer guided surgery using Nuclear Probes of the above type such that the said disadvantages of the known arrangements are avoided and that in particular the disadvantage of nuclear probes performing point measurements, the difficulty of the interpretation of the measurement data due to its fluctuation, the further disadvantage namely the need for many observations in order to get an idea of a valid measurement map in big areas and finally the disadvantage of the sequential process of measuring and then performing the surgical action are compensated and thus a more reliable operation is guaranteed.
This invention also compensates the particular disadvantage of the combination of nuclear probes with a camera related to the lack of the correlation of the measurement of the radioactivity to the anatomy by including tracking systems and a proper visualization.

The advantageous extending of the use of probes for combining position and orientation tracking with surface reconstruction, measurement and advanced visualization is also to be implemented.

In view of the above, a device according to claim 1 and a method according to claim 7 are provided. Particular embodiments of the invention are implemented by using Nuclear Probes of the above type and tracking systems and in particular by first generating an activity surface in three dimensions by synchronized recording of the radioactivity and the position and orientation of a nuclear probe and thus making possible the interpretation of the measurements as a composite result; second visualizing this composite result which allows the operator to get a smooth impression of the activity distribution and thus to compensate the fluctuation; third calculating a mesh by using the measured positions and the a priori knowledge that they lie on a surface and further generating a proper interpolation of the results based on the said mesh; and finally presenting the generated surface activity distribution to the surgeon when applying the therapeutic actions and thus allowing the bridging of the time gap between measurement and action.
In a preferred embodiment of the invention the generated surface activity distribution is presented using an augmented reality system. In it, firstly, a visual, colour encoded surface model is superimposed on the real image of an external camera or a laparoscope; secondly, based on the recorded activity and the position and orientation of any tracked therapeutic device in the same coordinate system as the one in which the nuclear probe is tracked, a radioactivity measurement is simulated turning the surgical instrument into a virtual probe, and allowing the guidance of the surgeon to the areas of interest during surgical procedures.
The new technology can be used in both invasive and minimally invasive surgery and provides an end-to-end solution for minimal resection of malign tumorous cells reducing the remaining residual and therefore the risks of reoccurrence. It also results in more precise detection and treatment of afflicted lymph nodes.
A further improvement is the generating the same auditory feedback one gets during the nuclear probe examination, while approaching the same tissue with surgical instruments during surgical procedures.
In addition this invention solves the additional disadvantage of the combination of nuclear probes with a camera related to the lack of the correlation of the measurement of the radioactivity to the anatomy by the synchronized recording of the radioactivity, the camera image and the position and orientation of a nuclear probe. Based on the said synchronized recording, an activity surface in three dimensions is generated and it is projected onto the real image of the camera as a colour-encoded surface. Thus the correlation of the measurement of the radioactivity to the anatomy is given in a proper visualization.

The invention will now be elucidated by reference to the embodiment partially illustrated schematically in the drawings.
Fig. 1: a schematic view of a tracked nuclear probe
Fig. 2: a schematic view of a tracked laparoscope
Fig. 3: a schematic view of a tracked surgical instrument
Fig. 4: a schematic view of an exemplary hardware set up for an experimental laparoscopic scan
Fig. 5: a schematic view of a laparoscopic scan on a human body with a laparoscope, a nuclear probe and a monitor
Fig. 6: schematic views of a body tissue, radioactive areas and a semi-transparent superposition of the radioactive areas to the body tissue
Fig. 7: a block diagram of a synchronized recording of the position and orientation measurement device
Fig. 8: presentation of a recorded activity by means of a tracked instrument
Fig. 9: schematic view of tracked combination of nuclear probe with camera

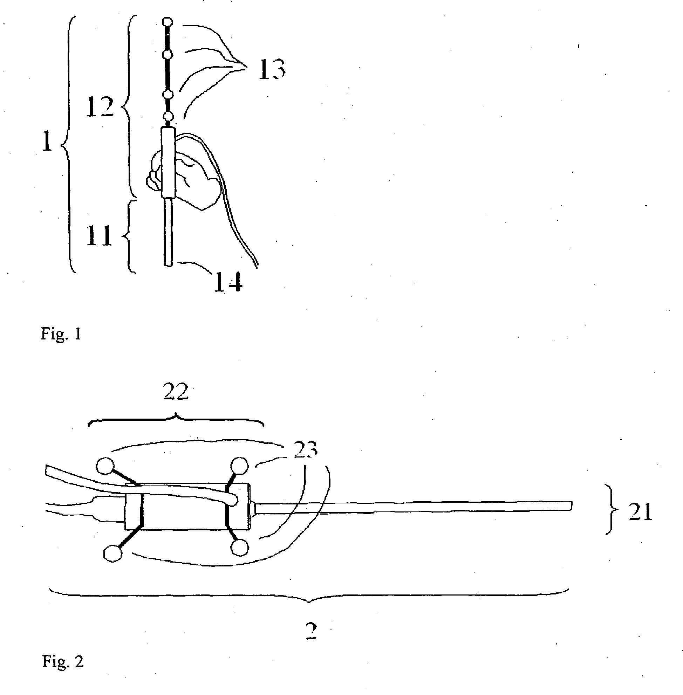

Fig. 1 shows a schematic of a tracked nuclear probe 1 which has essentially two parts, the first part is a nuclear probe 11 with a tip of nuclear probe 14 and the second part is an optical tracking target of nuclear probe 12 with an optical markers of nuclear probe 13. Based on the relative positions of the markers of nuclear probe 13, the position of the tip of the nuclear probe 14 and the probe axis can be estimated with a high precision in real time. The rotation around the axis of the nuclear probe 1 is not tracked, but it is also of no interest for the applications, since the measurement value is independent of this rotation. The readings of the nuclear probe 1 are sent to a proper data acquisition system for example a proper computer (not shown) using a data transfer means (not shown).

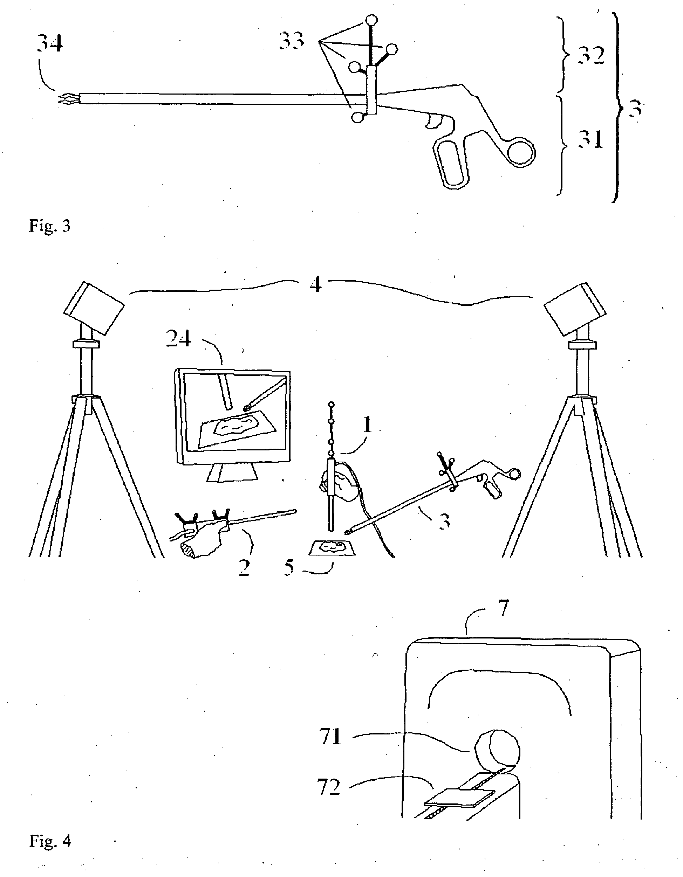

Fig. 2 shows a schematic view of a tracked instrument which is a tracked laparoscope 2 which has essentially two parts, the first part is a laparoscope 21 and the second part is an optical tracking target of laparoscope 22 which has optical markers of laparoscope 23. Based on the relative positions of the optical markers of laparoscope 23 the position and the orientation of laparoscope 21 can be estimated with a high precision in real time. Both the position and the orientation of laparoscope 21 are necessary to determine the correct view port of a laparoscopic video image 26, which is shown in Fig. 4.

Fig. 3 shows a schematic view of a tracked laparoscopic instrument 3 which is a tracked surgical instrument and has essentially two parts, the fist part is a laparoscopic instrument 31 with a tip of laparoscopic instrument 34 and the second part is an optical tracking target of laparoscopic instrument 32 with optical markers of laparoscopic instrument 33. Based on the relative positions of the markers of laparoscopic instrument 33, the position and orientation of the tip of the laparoscopic instrument 34 and axis of the laparoscopic instrument 31 can be estimated with a high precision in real time. In order to simulate the readings of the tracked nuclear probe 1 on the tip of laparoscopic instrument 34, both the position and the orientation of laparoscopic instrument 31 are necessary.

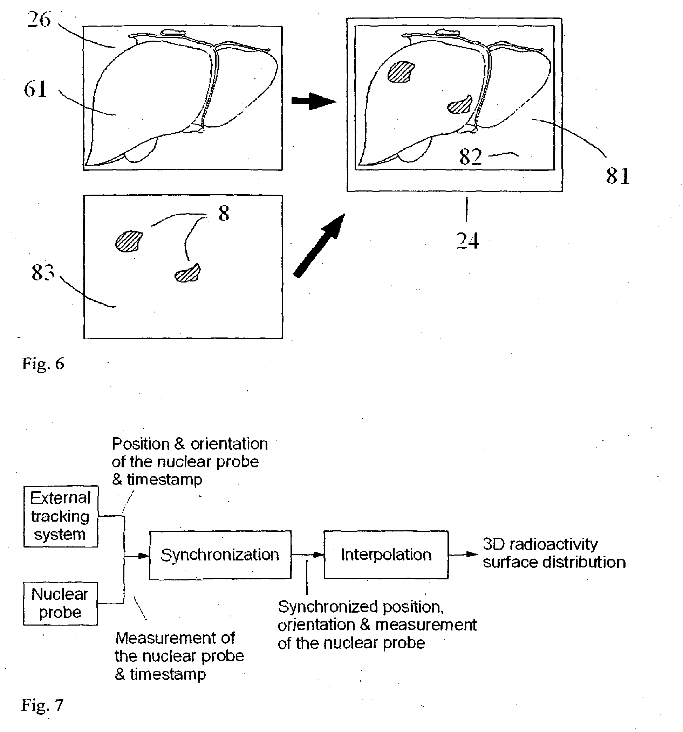

Fig. 4 shows an embodiment of a Device for 3D acquisition, 3D visualization and computer guided surgery using Nuclear Probes in an experimental case for a laparoscopic scan. A tracked nuclear probe 1 is used to acquire the 3D radioactive surface distribution 8 (shown in Fig. 6) of a phantom 5. An tracking system which can be for example an external optical tracking system 4 or an electro magnetic device (not shown) is used to track the tracked nuclear probe 1 as well as the tracked laparoscope 2 and the tracked laparoscopic instrument 3. The tracked laparoscope 2 acquires simultaneously a laparoscopic video image 26 (shown in Fig. 6). In order to visualized the acquired information a screen for image of laparoscope 24 is used. The said laparoscopic video image 26 is augmented with additional data like the 3D radioactive surface distribution 8. In case of the use of many cameras the video images of each of them can be augmented separately. A special case for that is for example a Head-Mounted Display where one image for each eye is augmented and thus a 3D impression is achieved. The tracked laparoscopic instrument 3 can also be tracked and the readings of the tracked nuclear probe 1 can be simulated on the tip of laparoscopic instrument 34. The simulated activity can be either displayed in the screen for image of laparoscope 24 or be fed back in form of acoustic signals that encode the 3D radioactive surface distribution 8. The encoding can be such that for a high radioactivity a high beep frequency and for a low radioactivity a low beep frequency is generated. A PET scanner 7 (Positron Emission Tomography) is used to validate the 3D radioactive surface distribution 8 in the phantom 5. Therefore the phantom 5 is positioned on a Phantom holder of PET scanner 72 and is to be moved into a gantry of PET scanner 71 to perform the imaging.

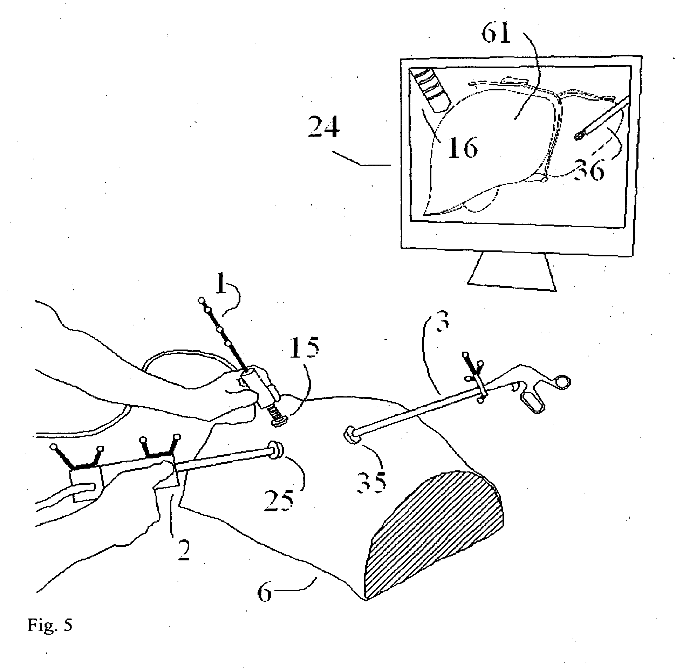

Fig. 5 shows a schematic view of a scan on a human body 6 with a tracked laparoscope 2, a tracked nuclear probe 1 and a screen for image of laparoscope 24 for scanning a body tissue 61. The tracked nuclear probe 1 is introduced through a proper trocar for tracked nuclear probe 15 into a human body 6 of a patient. The image of the inside is acquired using a tracked laparoscope 2 introduced by trocar for tracked laparoscope 25. Moreover the tracked laparoscopic instrument 3 can be introduced through a trocar for tracked laparoscopic instrument 35. A data acquisition system, for example a proper computer (not shown), integrates the laparoscopic video image 26 (shown in Fig. 6), the position and the orientation of laparoscope 21 and the position and orientation of the tip of the laparoscopic instrument 34 and axis of the laparoscopic instrument 31, where they are also synchronized with the readings of the tracked nuclear probe 1 and with the position and orientation of the tip of the nuclear probe 14. An image of the tip of nuclear probe on screen 16 as well as the body tissue 61 and an image of the tip of laparoscopic instrument 36 can be seen in the screen for image of laparoscope 24.

Fig. 6 shows schematic views of a body tissue 61, the laparoscopic video image 26, the 3D radioactive surface distribution 8, a projection of the said generated 3D radioactive surface distribution as a color encoded surface 83 and a semi-transparent superposition of 3D radioactive surface distribution 8 onto the body tissue 61 indicated as superimposed image 81. Additional information for example simulated radioactivity measurement 82 can be also shown on the screen for image of laparoscope 24. The simulated radioactivity measurement is calculated based on the 3D radioactive surface distribution 8 and the position and orientation of the tip of laparoscopic instrument 34. The simulated radioactivity measurement is to be equivalent to the one displayed by a nuclear probe 11 when detecting radioactivity.

Fig. 7 shows a block diagram of a synchronized recording of the position and orientation measurement device and the activity measures resulting in the generation of the three dimensional surface map that is presented to the surgeon. The external tracking system 4 feeds the data acquisition system with the position and orientation of the tip of the nuclear probe 14 as well as the timestamp of that measurement. Simultaneously the measurement of the nuclear probe 11 is also acquired with the timestamp of that measurement. Both the position and the orientation of the tip of the nuclear probe 14 and the measurement of the nuclear probe 11 are stored and synchronized. The synchronized position, orientation and measurement of the nuclear probe 11 are further interpolated to generate a 3D radioactive surface distribution 8.

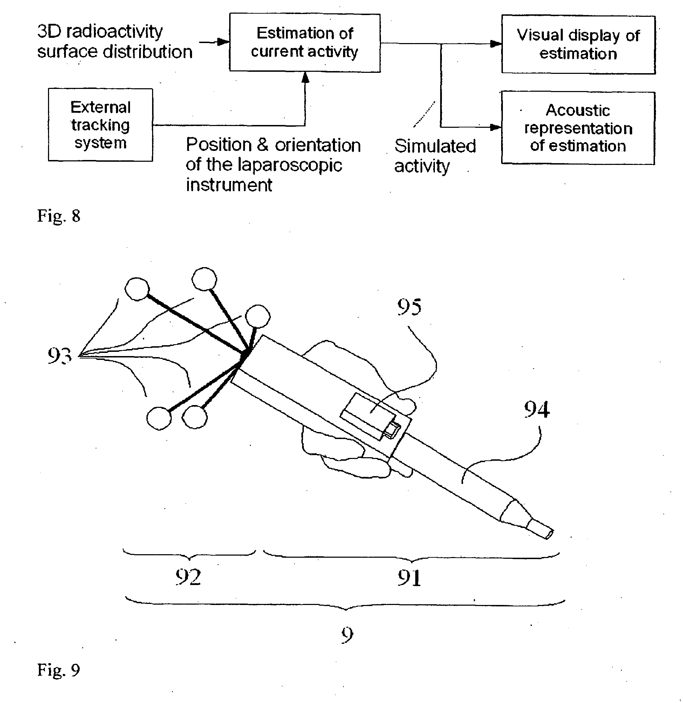

Fig. 8 shows a presentation of a recorded activity by means of a tracked laparoscopic instrument 3. This could result either in a visual presentation such as an augmented reality view for example as a superimposed image 81 or in an acoustic representation simulating the real sound or count visualization of the nuclear probe 11. The optical external tracking system 4 feeds the data acquisition system with the position and orientation of the tip of laparoscopic instrument 34. The 3D radioactive surface distribution 8 calculated as described in Fig. 7 can then be integrated to generate a simulated activity on the tip of laparoscopic instrument 34. Based on the simulated activity the proper visualization or acoustic feedback can be calculated.

Fig. 9 shows a preferred embodiment of a tracked combination of a nuclear probe with a camera 9 which has essentially two parts. The fist part is a combination of a nuclear probe with a camera 91 with a nuclear probe of combination of a nuclear probe with a camera 94 and an optical camera system, which can be for example only one camera of combination of a nuclear probe with a camera 95 or a light guide that transmits the optical image to a camera connected to the device (not shown). The second part is an optical tracking target of combination of a nuclear probe with a camera 92 with optical markers of combination of a nuclear probe with a camera 93. Based on the relative positions of the markers of combination of a nuclear probe with a camera 93, the position and orientation of the nuclear probe of combination of a nuclear probe with a camera 94 and the position and orientation of the optical camera system can be estimated with a high precision in real time. Based on the synchronized radioactivity measurements of the nuclear probe of combination of a nuclear probe with a camera 94 and the position and orientation of the nuclear probe of combination of a nuclear probe with a camera 94, a 3D radioactive surface distribution 8 can be also generated. With the 3D radioactive surface distribution 8 a projection of the generated 3D radioactive surface distribution as a colour encoded surface 83 can be generated using the viewing geometry of the optical camera system and shown also as a superimposed image 81.

### Reference list of drawings

1 tracked nuclear probe (modified beta-probe)
   11 nuclear probe
   12 optical tracking target of nuclear probe
   13 optical markers of nuclear probe
   14 tip of nuclear probe
   15 trocar for tracked nuclear probe
   16 image of the tip of nuclear probe on screen
2 tracked laparoscope
   21 laparoscope
   22 optical tracking target of laparoscope
   23 optical markers of laparoscope
   24 screen for image of laparoscope
   25 trocar for tracked laparoscope
   26 laparoscopic video image
3 tracked laparoscopic instrument
   31 laparoscopic instrument
   32 optical tracking target of laparoscopic instrument
   33 optical markers of laparoscopic instrument
   34 tip of laparoscopic instrument
   35 trocar for tracked laparoscopic instrument
   36 image of the tip of laparoscopic instrument
4 external optical tracking system
5 phantom
6 Human body
   61 body tissue
7 PET scanner
   71 Gantry of PET scanner
   72 Phantom holder of PET scanner
8 3D radioactive surface distribution
   81 superimposed image
   82 additional information for example simulated radioactivity measurement
   83 projection of the generated 3D radioactive surface distribution as a color encoded surface
9 tracked combination of a nuclear probe with a camera
   91 combination of a nuclear probe with a camera
   92 optical tracking target of nuclear probe of combination of a nuclear probe with a camera
   93 optical tracking markers of nuclear probe of combination of a nuclear probe with a camera
   94 nuclear probe of combination of a nuclear probe with a camera
   95 camera of combination of a nuclear probe with a camera

## Claims

1. A device for computer guided surgery comprising:
(a) a nuclear probe (11) with a tip (14), the nuclear probe being adapted for measuring radioactivity,
(b) a tracking system (4) for measuring a position and an orientation of said nuclear probe, and
(c) a data acquisition system for acquiring the position and orientation measurement and the radioactivity measurement of said nuclear probe in a coordinate system, and for acquiring respective timestamps of the measurements,
(d) an optical camera (95),
wherein said data acquisition system is adapted for storing and synchronizing the position and orientation measurement and the radioactivity measurement of said nuclear probe, **characterized in that** the device further comprises:
a module adapted for generating a 3D radioactive surface distribution (8) using the time synchronized position and orientation measurement and the radioactivity measurement of said nuclear probe; and
a module adapted for spatially registering a projection of the generated 3D radioactive surface distribution (8) with the image from the camera, and for visualizing the projected 3D radioactive surface distribution (8) in a superimposed image (81) in which an image from the camera is superimposed.

2. The device for computer guided surgery of claim 1, further comprising
a surgical instrument (21, 31);
a module for the measuring a position and orientation of the instrument (21, 31) in the same coordinate system as the nuclear probe (11); and
a module for the calculation and display of a simulated radioactivity measurement, the simulated radioactivity measurement being calculated based on the generated 3D radioactive surface distribution (8) and the measured position and orientation of the surgical instrument, and preferably
a module for feeding back the simulated radioactivity measurement in form of acoustic signals while navigating the surgical instrument over the generated 3D radioactive surface distribution (8).

3. The device for computer guided surgery of claim 1 or 2, further comprising a display for three dimensional visualization of said radioactive surface distribution (8).

4. The device for computer guided surgery of claim 2 or 3, wherein said tracking system (4) measures a position and an orientation of said camera, the camera being preferably a laparoscope.

5. The device for computer guided surgery of any one of the preceding claims, wherein the camera (95) and the nuclear probe are provided as a tracked combination, and the tracking system (4) is adapted for measuring a position and an orientation of said tracked combination.

6. The device for computer guided surgery of claim 2 or 5 further comprising, a system of coordinates, the position and the orientation of said nuclear probe in said system of coordinates, a surgical instrument, a position and an orientation of said surgical instrument in said system of coordinates,
whereby said surgical instrument is guided to said radioactive surface distribution during the surgical procedure.

7. A method for computer guided surgery, comprising:
(a) measuring a position, an orientation and a radioactivity measurement of a nuclear probe, **characterised in that**,
(b) acquiring respective time stamps of the measurements,
(c) synchronizing the measurements using the measurement time stamps,
(d) generating a radioactive surface distribution in three dimensions using the time synchronized position, orientation and radioactivity measurements of said nuclear probe;
(e) spatially registering a projection of the generated 3D radioactive surface distribution (8) with the viewing geometry of an optical camera, and
(f) visualizing the projected 3D radioactive surface distribution (8) in a superimposed image (81) in which an image of the camera is superimposed.

8. The method for computer guided surgery of claim 7, further comprising:
measuring a position and orientation of a surgical instrument in the same coordinate system as the nuclear probe; and
calculating a simulated radioactivity measurement based on the generated 3D radioactive surface distribution and the measured position and orientation of the surgical instrument, and displaying the calculated simulated radioactivity measurement, and preferably
feeding back the simulated radioactivity measurement in form of acoustic signals while navigating the surgical instrument over the generated 3D radioactive surface distribution.

9. The method for computer guided surgery of claim 7 or 8, further comprising:
visualizing said radioactive surface distribution in three dimensions,
calculating a mesh by using the measured positions of said nuclear probe and a priory condition that the measured positions must lie in the same surface;
generating an interpolation based on said radioactive surface distribution and said mesh; and
presenting the generated interpolation.

10. The method for computer guided surgery of any one of claims 7 to 9, further comprising:
(h) providing a system of coordinates,
(i) tracking the position and orientation of said nuclear probe in said system of coordinates,
(j) simulating a radioactivity measurement based on a measured radioactivity by a nuclear probe and a measured position and orientation of a surgical instrument in said system of coordinates,
whereby the surgical instrument can be used as virtual probe, and
whereby a surgeon is guided to areas of interest during surgical procedures.

11. The method for computer guided surgery of claim 10, further comprising:
(k) generating a visual display of estimation and/or an auditory feedback while approaching a tissue with said surgical instrument based on the simulated radioactivity measurement.

## Patentansprüche

1. Vorrichtung zur computergeführten Chirurgie, Folgendes umfassend:
(a) eine Nuklearsonde (11) mit einer Spitze (14), wobei die Nuklearsonde zum Messen von Radioaktivität angepasst ist,
(b) eine Nachführsystem (4) zum Messen einer Position und einer Ausrichtung der Nuklearsonde, und
(c) ein Datenerfassungssystem zum Erfassen des Positions- und Ausrichtungsmesswerts und des Radioaktivitätsmesswerts der Nuklearsonde in einem Koordinatensystem, und zum Erfassen jeweiliger Zeitstempel der Messwerte,
(d) eine optische Kamera (95),
wobei das Datenerfassungssystem zum Speichern und Synchronisieren des Positions- und Ausrichtungsmesswerts und des Radioaktivitätsmesswerts der Nuklearsonde angepasst ist, **dadurch gekennzeichnet, dass** die Vorrichtung darüber hinaus umfasst:
ein Modul, das dazu angepasst ist, eine 3D-Radioaktivitätsflächenverteilung (8) unter Verwendung des zeitsynchronisierten Positions- und Ausrichtungsmesswerts und des Radioaktivitätsmesswerts der Nuklearsonde zu generieren; und
ein Modul, das dazu angepasst ist, eine Projektion der generierten 3D-Radioaktivitätsflächenverteilung (8) mit dem Bild aus der Kamera räumlich in Übereinstimmung zu bringen und die projizierte 3D-Radioaktivitäts-flächenverteilung (8) in einem Überlagerungsbild (81) zu visualisieren, in dem ein Bild aus der Kamera überlagert ist.

2. Vorrichtung zur computergeführten Chirurgie nach Anspruch 1, darüber hinaus umfassend:
ein chirurgisches Instrument (21, 31);
ein Modul zum Messen einer Position und Ausrichtung des Instruments (21, 31) im selben Koordinatensystem wie die Nuklearsonde (11); und
ein Modul zum Berechnen und Anzeigen eines simulierten Radioaktivitätsmesswerts, wobei der simulierte Radioaktivitätsmesswert auf Grundlage der generierten 3D-Radioaktivitätsflächenverteilung (8) und der gemessenen Position und Ausrichtung des chirurgischen Instruments berechnet ist, und vorzugsweise
ein Modul zum Rückkoppeln des simulierten Radioaktivitätsmesswerts in Form von akustischen Signalen, während mit dem chirurgischen Instrument die generierte 3D-Radioaktivitätsflächenverteilung (8) überstrichen wird.

3. Vorrichtung zur computergeführten Chirurgie nach Anspruch 1 oder 2, darüber hinaus eine Anzeige zur dreidimensionalen Visualisierung der Radioaktivitätsflächenverteilung (8) umfassend.

4. Vorrichtung zur computergeführten Chirurgie nach Anspruch 2 oder 3, wobei das Nachführsystem (4) eine Position und Ausrichtung der Kamera misst, wobei es sich bei der Kamera vorzugsweise um ein Laparoskop handelt.

5. Vorrichtung zur computergeführten Chirurgie nach einem der vorhergehenden Ansprüche, wobei die Kamera (95) und die Nuklearsonde als Nachführkombination bereitgestellt sind und das Nachführsystem (4) dazu angepasst ist, eine Position und eine Ausrichtung der Nachführkombination zu messen.

6. Vorrichtung zur computergeführten Chirurgie nach Anspruch 2 oder 5, darüber hinaus ein System von Koordinaten, die Position und die Ausrichtung der Nuklearsonde in dem System von Koordinaten, ein chirurgisches Instrument, eine Position und eine Ausrichtung des chirurgischen Instruments in dem System von Koordinaten umfassend,
wobei das chirurgische Instrument während des chirurgischen Prozesses zur Radioaktivitätsflächenverteilung geführt wird.

7. Verfahren zur computergeführten Chirurgie, Folgendes umfassend:
(a) Messen einer Position, einer Ausrichtung und eines Radioaktivitätsmesswerts einer Nuklearsonde,
**dadurch gekennzeichnet, dass**
(b) jeweilige Zeitstempel der Messwerte erfasst werden,
(c) die Messwerte anhand der Messwertzeitstempel synchronisiert werden,
(d) eine Radioaktivitätsflächenverteilung in drei Dimensionen unter Verwendung der zeitsynchronisierten Position, Ausrichtung und des Radioaktivitätsmesswerts der Nuklearsonde generiert wird;
(e) eine Projektion der generierten 3D-Radioaktivitätsflächenverteilung (8) mit der Aufnahmegeometrie einer optischen Kamera räumlich in Übereinstimmung gebracht wird, und
(f) die projizierte 3D-Radioaktivitätsflächenverteilung (8) in einem Überlagerungsbild (81) visualisiert wird, in dem ein Bild der Kamera überlagert ist.

8. Verfahren zur computergeführten Chirurgie nach Anspruch 7, darüber hinaus umfassend:
Messen einer Position und Ausrichtung eines chirurgischen Instruments im selben Koordinatensystem wie die Nuklearsonde; und
Berechnen eines simulierten Radioaktivitätsmesswerts auf Grundlage der generierten 3D-Radioaktivitätsflächenverteilung und der gemessenen Position und Ausrichtung des chirurgischen Instruments, und Anzeigen des berechneten simulierten Radioaktivitätsmesswerts, und vorzugsweise
Rückkoppeln des simulierten Radioaktivitätsmesswerts in Form von akustischen Signalen, während mit dem chirurgischen Instrument die generierte 3D-Radioaktivitätsflächenverteilung überstrichen wird.

9. Verfahren zur computergeführten Chirurgie nach Anspruch 7 oder 8, darüber hinaus umfassend:
Visualisieren der Radioaktivitätsflächenverteilung in drei Dimensionen,
Berechnen eines Gitters unter Verwendung der gemessenen Positionen der Nuklearsonde und einer vorrangigen Bedingung, dass die gemessenen Positionen in derselben Fläche liegen müssen;
Generieren einer Interpolation auf Grundlage der Radioaktivitätsflächenverteilung und des Gitters; und
Darstellen der generierten Interpolation.

10. Verfahren zur computergeführten Chirurgie nach einem der Ansprüche 7 bis 9, darüber hinaus umfassend:
(h) Bereitstellen eines Systems von Koordinaten,
(i) Nachführen der Position und Ausrichtung der Nuklearsonde in dem System von Koordinaten,
(j) Simulieren einer Radioaktivitätsmessung auf Grundlage einer gemessenen Radioaktivität durch eine Nuklearsonde und einer gemessenen Position und Ausrichtung eines chirurgischen Instruments in dem System von Koordinaten,
wobei das chirurgische Instrument als virtuelle Sonde verwendet werden kann, und
wobei der Chirurg während chirurgischer Prozesse zu Bereichen von Interesse geführt wird.

11. Verfahren zur computergeführten Chirurgie nach Anspruch 10, darüber hinaus umfassend:
(k) Generieren einer visuellen Schätzanzeige und/oder einer hörbaren Rückkopplung auf Grundlage der simulierten Radioaktivitätsmessung bei der Annäherung an ein Gewebe mit dem chirurgischen Instrument.

## Revendications

1. Dispositif de chirurgie guidée par ordinateur, comprenant :
(a) une sonde nucléaire (11) munie d'une pointe (14), la sonde nucléaire étant apte à mesurer la radioactivité,
(b) un système de poursuite (4) destiné à mesurer une position et une orientation de ladite sonde nucléaire, et
(c) un système d'acquisition de données destiné à acquérir la mesure de position et d'orientation et la mesure de radioactivité de ladite sonde nucléaire dans un système de coordonnées, et à acquérir des estampilles temporelles respectives des mesures,
(d) une caméra optique (95),
ledit système d'acquisition de données étant apte à stocker et à synchroniser la mesure de position et d'orientation et la mesure de radioactivité de ladite sonde nucléaire, **caractérisé en ce que** le dispositif comprend en outre :
un module apte à générer une distribution de surface radioactive 3D (8) au moyen de la mesure de position et d'orientation synchronisée dans le temps et de la mesure de radioactivité de ladite sonde nucléaire ; et
un module apte à enregistrer spatialement une projection de la distribution de surface radioactive 3D (8) avec l'image provenant de la caméra, et à visualiser la distribution de surface radioactive 3D (8) projetée dans une image superposée (81) dans laquelle une image provenant de la caméra est superposée.

2. Le dispositif de chirurgie guidée par ordinateur de la revendication 1, comprenant en outre
un instrument chirurgical (21, 31) ;
un module destiné à mesurer une position et une orientation de l'instrument (21, 31) dans le même système de coordonnées que la sonde nucléaire (11);et
un module pour le calcul et l'affichage d'une mesure de radioactivité simulée, la mesure de radioactivité simulée étant calculée sur la base de la distribution de surface radioactive 3D (8) générée et de la position et de l'orientation mesurées de l'instrument chirurgical, et de préférence
un module destiné à informer en retour de la mesure de radioactivité simulée sous forme de signaux acoustiques pendant la navigation de l'instrument chirurgical sur la distribution de surface radioactive 3D (8) générée.

3. Le dispositif de chirurgie guidée par ordinateur de la revendication 1 ou 2, comprenant en outre un affichage pour la visualisation en trois dimensions de ladite distribution de surface radioactive (8).

4. Le dispositif de chirurgie guidée par ordinateur de la revendication 2 ou 3, dans lequel ledit système de poursuite (4) mesure une position et une orientation de ladite caméra, la caméra étant de préférence un laparoscope.

5. Le dispositif de chirurgie guidée par ordinateur de l'une quelconque des revendications précédentes, dans lequel la caméra (95) et la sonde nucléaire sont fournies comme combinaison poursuivie, et le système de poursuite (4) est apte à mesurer une position et une orientation de ladite combinaison poursuivie.

6. Le dispositif de chirurgie guidée par ordinateur de la revendication 2 ou 5, comprenant en outre un système de coordonnées, la position et l'orientation de ladite sonde nucléaire dans ledit système de coordonnées, un instrument chirurgical, une position et une orientation dudit instrument chirurgical dans ledit système de coordonnées,
ledit instrument chirurgical étant guidé vers ladite distribution de surface radioactive pendant l'opération chirurgicale.

7. Procédé de chirurgie guidée par ordinateur, comprenant :
(a) la mesure d'une position, d'une orientation et une mesure de radioactivité d'une sonde nucléaire,
**caractérisée par**
(b) l'acquisition d'estampilles temporelles respectives des mesures,
(c) la synchronisation des mesures au moyen des estampilles temporelles de mesure,
(d) la génération d'une distribution de surface radioactive en trois dimensions au moyen des mesures de position, d'orientation et de radioactivité synchronisées dans le temps de ladite sonde nucléaire ;
(e) l'enregistrement spatial d'une projection de la distribution de surface radioactive 3D (8) générée avec la géométrie de visée d'une caméra optique, et
(f) la visualisation de la distribution de surface radioactive 3D (8) projetée dans une image superposée (81) dans laquelle une image de la caméra est superposée.

8. Le procédé de chirurgie guidée par ordinateur de la revendication 7, comprenant en outre :
la mesure d'une position et d'une orientation d'un instrument chirurgical dans le même système de coordonnées que la sonde nucléaire ; et
le calcul d'une mesure de radioactivité simulée sur la base de la distribution de surface radioactive 3D générée et de la position et de l'orientation mesurées de l'instrument chirurgical, et l'affichage de la mesure de radioactivité simulée calculée, et de préférence
l'information en retour de la mesure de radioactivité simulée sous forme de signaux acoustiques pendant la navigation de l'instrument chirurgical sur la distribution de surface radioactive 3D générée.

9. Le procédé de chirurgie guidée par ordinateur de la revendication 7 ou 8, comprenant en outre :
la visualisation de ladite distribution de surface radioactive en trois dimensions,
le calcul d'un réseau au moyen des positions mesurées de ladite sonde nucléaire et à une condition préalable que les positions mesurées doivent se situer dans la même surface ;
la génération d'une interpolation sur la base de ladite distribution de surface radioactive et dudit réseau ; et
la présentation de l'interpolation générée.

10. Le procédé de chirurgie guidée par ordinateur de l'une quelconque des revendications 7 à 9, comprenant en outre :
(h) la fourniture d'un système de coordonnées,
(i) la poursuite de la position et de l'orientation de ladite sonde nucléaire dans ledit système de coordonnées,
(j) la simulation d'une mesure de radioactivité sur la base d'une radioactivité mesurée par une sonde nucléaire et d'une position et d'une orientation mesurées d'un instrument chirurgical dans ledit système de coordonnées,
l'instrument chirurgical pouvant être utilisé comme sonde virtuelle, et un chirurgien étant guidé vers des zones d'intérêt pendant des opérations chirurgicales.

11. Le procédé de chirurgie guidée par ordinateur de la revendication 10, comprenant en outre :
(k) la génération d'un affichage visuel d'estimation et/ou d'un retour d'information auditif pendant l'approche d'un tissu avec ledit instrument chirurgical sur la base de la mesure de radioactivité simulée.
